# EUROPÄISCHE PATENTSCHRIFT

(11) **EP 1 279 409 B1**
(45) Veröffentlichungstag und Bekanntmachung des Hinweises auf die Patenterteilung: **23.12.2009**
(21) Anmeldenummer: 02015704.6
(22) Anmeldetag: 12.07.2002
(51) Int. Cl.: A61M 1/16, A61M 39/10, A61M 39/14, B01F 3/08

(54) **Verfahren und Vorrichtung sowie Konnektor und Konzentratbehältereinheit zur Zubereitung von Lösungen**
Method and device, as well as connector and container for concentrate, for the preparation of solutions
Méthode et dispositif, ainsi que connecteur et conteneur pour le concentré, pour la préparation de solutions

(30) Priorität: 25.07.2001 DE 10136262
(43) Veröffentlichungstag der Anmeldung: 29.01.2003
(73) Patentinhaber: Fresenius Medical Care Deutschland GmbH, 61352 Bad Homburg v.d.H. (DE)
(72) Erfinder: Brandl, Matthias, Dr., 97631 Bad Königshofen (DE); Hilgers, Peter, Dr., 97453 Schonungen (DE); Kugelmann, Franz, Dr., 66606 St. Wendel (DE); Meisinger, Matthias, 66583 Spiesen-Elversberg (DE)
(74) Vertreter: Laufhütte, Dieter

(56) Entgegenhaltungen:
- EP-A- 0 197 553
- DE-A- 19 605 260
- US-A- 4 161 949
- US-A- 4 338 933
- US-B1- 6 234 538

## Beschreibung

Die vorliegende Erfindung betrifft ein Verfahren sowie eine Vorrichtung zur Zubereitung von aus wenigstens zwei Konzentraten und einem Lösungsmittel herzustellenden Lösungen sowie einen Konnektor und eine ein Konzentrat enthaltende Konzentratbehältereinheit.

Eine wichtige Anwendungsmöglichkeit für ein derartiges Verfahren bzw. eine derartige Vorrichtung ist die Dialyse, bei der hochreine Dialysierflüssigkeiten benötigt werden, die in auf den Patienten abgestimmten Zusammensetzungen und Konzentrationen bereitgestellt werden müssen. Die Dialysierflüssigkeiten werden üblicherweise aus zwei Konzentraten hergestellt, die in getrennten Behältnissen aufbewahrt werden und erst im Rahmen der Herstellung der Dialysierflüssigkeit, vermischt werden.

Bei der Herstellung von Dialysierflüssigkeiten ist unbedingt sicherzustellen, daß keine Fehler unterlaufen, da fehlerhafte Zusammensetzungen der Dialysierflüssigkeit oder fehlerhafte Konzentrationen der darin enthaltenen Komponenten zu einer erheblichen Gefährdung des im Rahmen der Dialyse zu behandelnden Patienten führen können.

Es sind Batch-Systeme bekannt, bei denen das die Dialysierflüssigkeit bereitstellende medizinische Personal in ein Einspülgefäß die unterschiedlichen, zur Herstellung benötigten Konzentrate einfüllt. Anschließend werden diese von vorgewärmtem, hochreinem Wasser gelöst bzw. mit diesem vermischt und in ein Vorlagegefäß gespült, das anschließend an den Ort der durchzuführenden Behandlung verbracht wird. Zwar kann die Kontrolle der fertigen Dialysierflüssigkeit durch eine Leitfähigkeitsmessung erfolgen, jedoch sind Fehler bei der Zubereitung der Dialysierflüssigkeit insbesondere dann nicht auszuschließen, wenn die fehlerhafte und die gewünschte Lösung ähnliche Leitfähigkeitswerte aufweisen.

Aus der DE 196 05 260 A ist ein Verfahren zur Zubereitung einer Lösung aus zwei Konzentraten bekannt, bei welchen die Konzentrate aus Flaschen, welche mit Chemikalienapplikatoren verbunden werden, in einem Behälter zusammenfließen. Die Chemikalienapplikatoren stehen dabei mit dem Behälter in kommunizierender Verbindung, so dass die Konzentrate nach dem Durchstoßen der Membran durch einen Aufstoßdorn über eine schräge Platte in den Behälter fließen. Bei diesem vorbekannten Verfahren erfolgt die Konnektierung der einzelnen Konzentratbehältnisse und die Steuerung des Lösungsmittelflusses unabhängig voneinander. Dieser Verfahrensablauf, bei dem eine Vielzahl von Ventilen eingesetzt und angesteuert werden müssen, ist kompliziert.

Aus der US-A-4 161 949 sowie aus der US-B1-6 234 538 sind jeweils aseptische Konnektierungen bekannt, bei welchen lediglich eine erste Leitung mit einer zweiten Leitung verbunden ist.

Aus der US-A-4 338 933 ist ein Konnektor für die Peritonealdialyse bekannt, durch welchen vor- und zurückströmende Flüssigkeit in verschiedene Schläuche geleitet werden kann.

Es ist die Aufgabe der vorliegenden Erfindung, ein Verfahren und eine Vorrichtung zur Zubereitung von aus wenigstens zwei Konzentraten und einem Lösungsmittel herzustellenden Lösung dahingehend weiterzubilden, daß es vereinfacht wird und das Auftreten von Fehlern bei der Zubereitung der Lösung weiter vermindert wird. Diese Aufgabe wird durch ein Verfahren mit den Merkmalen des Patentspruchs 1, durch eine Vorrichtung mit den Merkmalen des Patentanspruch 4 bzw. durch einen Konnektor gemäß Patentanspruch 10 und eine Konzentratbehältereinheit nach Patentanspruch 11 gelöst.

Bei dem erfindungsgemäßen Verfahren erfolgt nach dem Einlegen von Konzentratbehältnissen in zugehörigen Aufnahmen die Verbindung eines ersten Konzentratbehältnesses mit einer Lösungsmittelquelle, anschließend wird das erste Konzentratbehältnis vom Lösungsmittel durchströmt und die Lösung in ein Sammelbehältnis geführt. Nach dem Ausspülen des Konzentrats aus dem ersten Konzentratbehältnis erfolgt eine automatische Konnektierung eines zweiten Konzentratbehältnisses, woraufhin das zweite Konzentratbehältnis vom Lösungsmittel durchströmt und die Lösung ebenfalls dem Sammelbehältnis zugeführt wird. In einer ersten Zubereitungsphase erfolgt die Durchströmung und damit die Auflösung des Konzentrates ausschließlich durch das erste Konzentratbehältnis. Nach der automatischen Konnektion des zweiten Konzentratbehältnisses wird dieses durchströmt. Die darin enthaltenen Stoffe werden aufgelöst und ebenfalls ausgespült.

Die vorliegende Erfindung erniedrigt das Fehlbedienungsrisiko durch Automatisierung der Konzentratzugabe. Nach dem Ausspülen des Konzentrates aus dem ersten Konzentratbehältnis wird automatisch auf das zweite Konzentratbehältnis geschaltet, woraufhin dieses durchströmt wird und die Lösung ebenfalls dem Sammelbehältnis zugeführt wird. Der Bedienungskomfort bei einem derartigen Verfahren ist aufgrund der einfachen Handhabung, d. h. der Minimierung von Benutzerschritten, sehr hoch. Benutzerinteraktionen während der Zubereitungszeit werden vermieden.

Das Lösungsmittel kann durch Reversosmose hergestelltes hochreines Wasser sein. In dem ersten Konzentratbehältnis kann ein NaCl, NaHCO₃ und Glucose enthaltenes Trockenkonzentrat und in dem zweiten Konzentratbehältnis kann ein flüssiges Konzentrat vorliegen, das als Hauptbestandteile die Ionen K⁺, Ca²⁺, Mg²⁺ sowie NaCl enthält. Auf diese Weise läßt sich zuverlässig ultrareine Dialysierflüssigkeit herstellen.

In weiterer Ausgestaltung der vorliegenden Erfindung ist vorgesehen, daß die automatische Konnektierung des zweiten Konzentratbehältnisses erfolgt, wenn eine definierte Zeitspanne seit Beginn der Durchströmung des ersten Konzentratbehältnisses abgelaufen ist, wenn die Leitfähigkeit der das erste Konzentratbehältnis verlassenden Lösung einen Grenzwert unterschritten hat oder wenn der Füllstand in dem Sammelbehältnis einen vorgegebenen Wert übersteigt. Entsprechend ist in diesem Fall eine Steuereinheit vorgesehen, die einen Abgleich zwischen dem entsprechenden Istwert und einem Soll- bzw. Grenzwert vornimmt und bei Erreichen des Sollwertes bzw. Grenzwertes die automatische Konnektierung des zweiten Konzentratbehälntisses veranlaßt.

In weiterer Ausgestaltung der vorliegenden Erfindung ist vorgesehen, daß gleichzeitig mit der automatischen Konnektierung des zweiten Konzentratbehältnisses eine automatische Flußumleitung des ersten Konzentratbehältnisses erfolgt.

Aufgrund der eindeutig vorgegebenen Reihenfolge der Konzentratzugabe, ist eine Verwechslung ausgeschlossen. Darüber hinaus kann nicht der Fall eintreten, daß versehentlich mehrfach das eine oder mehrfach das andere Konzentrat zugegeben wird. Auch kann der Fall nicht eintreten, daß versehentlich eines der Konzentrate weggelassen wird, da die vorliegende Erfindung sicherstellt, daß nach dem Ausspülen des Konzentrats aus dem ersten Konzentratbehältnis eine automatische Konnektierung und anschließende Durchspülung des zweiten Konzentratbehältnisses erfolgt.

Die vorliegende Erfindung betrifft ferner eine Vorrichtung zur Zubereitung von aus wenigstens zwei Konzentraten und einem Lösungsmittel herzustellenden Lösungen. Die Vorrichtung weist wenigstens zwei Konzentratbehältnisse auf, sowie Zufuhrleitungen, durch die das Lösungsmittel zu den Konzentratbehältnissen führbar ist, Ablaufleitungen, mittels derer die Lösungen von den Konzentratbehältnissen in ein Sammelbehältnis führbar sind, einen Konnektor, der in einer ersten Position eine Lösungsmittelquelle mit dem ersten Konzentratbehältnis und in einer zweiten Position die Lösungsmittelquelle mit dem zweiten Konzentratbehältnis verbindet, sowie schließlich Mittel, durch die der Konnektor von der ersten in die zweite Position bewegbar ist. Nach dem Einlegen der Konzentratbehältnisse in die erfindungsgemäße Vorrichtung bzw. in die dafür vorgesehenen Aufnahmen der Vorrichtung erfolgt die zeitliche Abfolge sowie die Konnektion und die Flußregulierung automatisch. Die Aufgabe des Benutzers reduziert sich dementsprechend auf das Einlegen der gewünschten Konzentratbehältnisse.

Ein in zwei Positionen bewegbarer Konnektor ist beispielsweise aus der EP 0 197 553 A2 bekannt. Der darin offenbarte Konnektor findet bei der Peritonealdialyse Anwendung und ermöglicht je nach Konnektorposition den Fluß von einem Dialysebeutel zu dem Patienten, in einen Leerbeutel oder auch den Fluß von dem Patienten in den Leerbeutel. Der Konnektor weist ein weibliches Konnektorstück auf, das über eine Leitung mit einem Peritonealkatheder verbunden ist. Dieses Konnektorstück kann mit einem männlichen Konnektorstück verbunden werden, um das Einlassen von frischer Dialysierflüssigkeit aus einem vollen Beutel in den Peritonealraum zu ermöglichen. Über eine weitere Leitung ist ein Leerbeutel mit dem männlichen Konnektorstück verbunden. Das weibliche Konnektorstück weist ein Absperrorgan auf, welches bei einem vorbestimmten Konnektionszustand des Konnektors von einem zentralen Rohrstück des männlichen Konnektorstücks geöffnet wird. Durch die Anordnung eines zweiten Anschlusses am männlichen Konnektorstück wird in Zusammenwirkung mit dem als Membran ausgeführten Absperrorgan des weiblichen Konnektorstückes und einer radialen Öffnung im zentralen Rohrstück eine Fluidverbindung mit beiden Beuteln und dem Peritonealraum ermöglicht.

Besonders vorteilhaft ist es, wenn die Konzentratbehältnisse als Einmalartikel ausgeführt sind und mit jeweils zwei selbstdichtenden Konnektorelementen versehen sind. Die Verwendung von Einmalartikeln bringt den Vorteil mit sich, daß diese kostengünstig sind. Aufgrund der selbstdichtenden Anschlüsse sind die Konzentratbehältnisse vor und während der Anwendung verschlossen, wodurch Anwendungsfehler sowie eine falsche Lösungszubereitung verhindert wird und Keimfreiheit der herzustellenden Dialysierflüssigkeit gewährleistet wird. Jeder der Konzentratbehältnisse weist jeweils zwei Konnektorelemente auf, die je nach Anordnung den Zufluß in die Konzentratbehältnisse bzw. den Ablauf aus den Konzentratbehältnissen ermöglichen.

In weiterer Ausgestaltung der vorliegenden Erfindung ist vorgesehen, daß die Konzentratbehältnisse trichterförmig ausgeführt sind. Ein derartiges Design der Behälter ermöglicht die optimale und vollständige Auflösung der Konzentrate und zudem in entsprechender Verbindung mit einer Ablaufleitung die vollständige Entleerung der Konzentratbehältnisse. Dadurch ergibt sich der Vorteil, daß in den Einmalartikeln allenfalls ein minimales Restvolumen nach Gebrauch verbleibt.

In weiterer Ausgestaltung der vorliegenden Erfindung ist vorgesehen, daß der Konnektor ein mit einem ersten Konzentratbehältnis in Verbindung stehendes erstes Konnektorelement aufweist, in das ein mit einem zweiten Konzentratbehältnis in Verbindung stehendes zweites Konnektorelement einführbar ist, und daß in dem ersten Konnektorelement ein Aufstoßdorn vorgesehen ist, mittels dessen bei der Bewegung des Konnektors in die zweiten Position eine das zweite Konnektorelement verschließende Membran durchtrennbar ist. Zusätzlich ist vorgesehen, daß das erste Konnektorelement eine dieses abschließende Membran aufweist, vorzugsweise eine Silikonmembran. Vor der Durchströmung des zweiten Konzentratbehältnisses sind die Membranen unversehrt und es wird ausschließlich das erste Konzentratbehältnis durchströmt. Beim Konnektionsvorgang wird zunächst die genannte Silikonmembran durchstoßen. Damit wird zunächst eine dichtende Verbindung zum zweiten Konzentratbehältnis hergestellt. Schließlich wird in einer weiteren Phase des Konnektionsvorgangs die das zweite Konnektorelement verschließende Membran durch den Aufstoßdorn durchstoßen, was dazu führt, daß der Fluß des Lösungsmittels nunmehr hauptsächlich durch das zweite Konzentratbehältnis geführt wird. Dabei ist es günstig aber nicht notwendig, daß der Fluß durch das erste Konzentratbehältnis vollständig unterbunden wird.

In bevorzugter Ausgestaltung der vorliegenden Erfindung ist eine Anschlußleitung vorgesehen, die an dem ersten Konnektorteil angeordnet ist und durch die das Lösungsmittel von der Lösungsmittelquelle zum Konnektor bzw. die Lösung vom Konnektor zum Sammelbehältnis führbar ist.

Um den Aufstoßdorn des ersten Konnektorelements kann sich ein Ringraum erstrecken, der einen Teil der Zufuhrleitung bzw. Abfuhrleitung bildet, die den Konnektor mit dem ersten Konzentratbehältnis verbindet. Vor der Konnektierung wird das Lösungsmittel durch den Aufstoßdorn und anschließend durch den Ringraum geführt, der einen ersten Teil der Zufuhrleitung bildet, die zu dem ersten Konzentratbehältnis führt. Nach der Konnektion wird der Ringraum durch das zweite Konnektorelement ausgefüllt und das Lösungsmittel nun durch den Aufstoßdorn durch das zweite Konnektorelement in das zweite Konzentratbehältnis geführt.

Die vorliegende Erfindung betrifft ferner einen Konnektor mit einem ersten Konnektorelement, das mit einem ersten Konzentratbehältnis in Verbindung steht oder verbindbar ist, und mit einem zweiten Konnektorelement, das mit einem zweiten Konzentratbehältnis in Verbindung steht oder verbindbar ist, wobei das erste Konnektorelement von einer Membran verschlossen ist, die durch das in das erste Konnektorelement einführbare zweite Konnektorelement durchtrennbar ist und wobei das erste Konnektorelement einen Aufstoßdorn aufweist, mittels dessen eine das zweite Konnektorelement verschließende Membran durchtrennbar ist.

Die vorliegende Erfindung betrifft ferner eine Konzentratbehältereinheit mit einem in einem Konzentratbehältnis aufgenommenen festen oder flüssigen Konzentrat sowie mit zwei ersten oder zwei zweiten Konnektorelementen, wobei die ersten Konnektorelemente eine diese verschließende Membran sowie einen mit einer Anschlußleitung in Verbindung stehenden Aufstoßdorn aufweisen, der von einem mit dem Konzentratbehältnis in Verbindung stehenden Ringraum umgeben ist und wobei die zweiten Konnektorelemente mit dem Konzentratbehältnis in Verbindung stehen und eine die zweiten Konnektorelemente verschließende Membran aufweisen. Die Konzentratbehältereinheit ist vorteilhaft als Einmalartikel ausgeführt.

Die Konzentratbehältereinheit kann eine Zu- und Ablaufleitung umfassen, die sich von den ersten und zweiten Konnektorelementen zu dem Konzentratbehältnis bzw. von diesem zu den ersten und zweiten Konnektorelementen erstrecken.

In weiterer Ausgestaltung der vorliegenden Erfindung ist vorgesehen, daß die das erste und/oder das zweite Konnektorelement verschließende Membran eine Silikonmembran ist.

Das Konzentrat kann ein NaCl, NaHO₃ und Glucose enthaltendes Trockenkonzentrat oder ein flüssiges Konzentrat sein, das als Hauptbestandteile die Ionen K⁺, Ca²⁺, Mg²⁺ sowie NaCl enthält. Das flüssige Konzentrat kann ferner die Bestandteile Citrat und HCl enthalten.

Weitere Einzelheiten und Vorteile werden anhand eines in der Zeichnung dargestellten Ausführungsbeispiels näher erläutert. Es zeigen:
- Fig. 1:: eine schematische Darstellung des Flußes durch ein erstes Konzentratbehältnis mit dem Konzentrat DS in einer ersten Füllphase und anschließend durch ein zweites Konzentratbehältnis mit dem Konzentrat HC in einer zweiten Füllphase,
- Fig. 2:: eine schematische Darstellung des Flußes durch das erste Konzentratbehältnis unmittelbar vor der Konnektion des zweiten Konzentratbehältnisses,
- Fig. 3:: eine schematische Darstellung des Aufbaus des Konnektors mit erstem und zweitem Konnektorelement,
- Fig. 4:: Darstellungen unterschiedlicher Konnektionspositionen vor und nach der Konnektion des zweiten Konzentratbehältnisses und
- Fig. 5:: eine Konnektoranordnung gemäß Fig. 4 in horizontaler Ausführung.

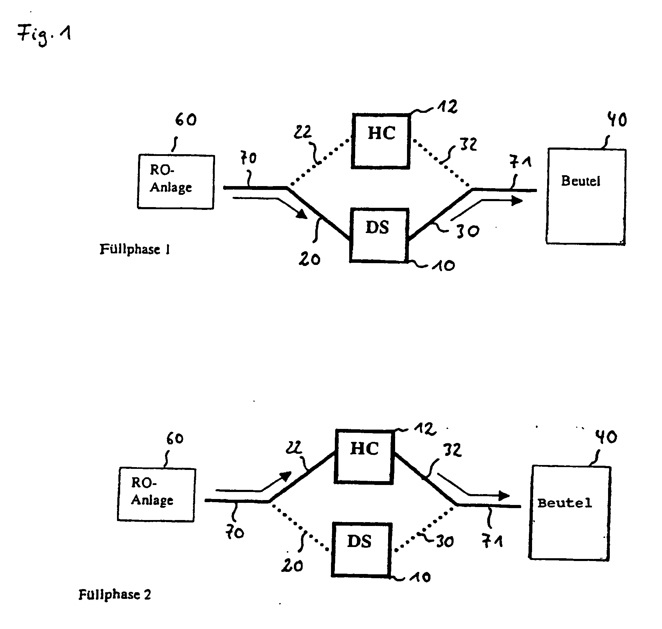

Fig. 1 zeigt in der oberen Darstellung die sogenannte Füllphase 1, in der hochreines Wasser aus der Lösungsmittelquelle 60 (RO-Anlage) in das erste Konzentratbehältnis 10 geführt wird. In diesem befindet sich das Trockenkonzentrat DS, das NaCl, NaHCO₃ und Glucose enthält. Die beiden Konzentratbehältnisse 10, 12 sind mit Zufuhrleitungen 20, 22 versehen, die im Anschluß an die Anschlußleitung 70 durchströmt werden und durch die das Lösungsmittel zu den Konzentratbehältnissen 10, 12 führbar ist. Ferner sind Ablaufleitungen 30, 32 vorgesehen, mittels derer die Lösungen von den Konzentratbehältnissen 10, 12 über eine Anschlußleitung 71 in ein Sammelbehältnis 40 geführt werden. Die das erste Konzentratbehältnis 10 verlassende Lösung wird einem Sammelbehältnis 40 zugeführt, das vorliegend als Beutel ausgeführt ist. Zu einem vorgegebenen Zeitpunkt erfolgt die automatische Konnektierung des zweiten Konzentratbehältnisses 12, in dem das flüssige Konzentrat HC vorliegt, das als Hauptbestandteile die Ionen K⁺, Ca²⁺, Mg²⁺ sowie NaCl enthält. Zusätzlich können hierin Citrat und HCl vorhanden sein. Das Lösungsmittel strömt nunmehr von der Lösungsmittelquelle 60 durch das zweite Konzentratbehältnis 12 in das Sammelbehälters 40. Dieser Zustand ist als Füllphase 2 in Fig. 1, unten dargestellt.

Die beiden Konzentratbehältnisse 10, 12 sind als kostengünstige Einmalartikel (Disposable) konzipiert. Die Konzentratbehältnisse sind jeweils mit zwei Konnektorelementen versehen, die selbstdichtend konnektieren. Die Konzentratbehältnisse 10, 12 sind trichterförmig ausgestaltet und erlauben aufgrund der Anordnung einer Ablaufleitung im unteren Bereich des Trichters eine weitgehend vollständige Entleerung und damit eine optimale Ausnutzung der Konzentrate.

Die Konzentratbehältnisse 10, 12 sind vor und während der Anwendung verschlossen, verhindern somit Anwendungsfehler und Fehler bei der Lösungszubereitung und garantieren die keimfreie Herstellung der Dialysierflüssigkeit.

Ein weiterer Vorteil des vorliegenden Verfahrens ist, daß zur Flußregulierung keine Ventile eingesetzt werden müssen, da aufgrund der Konnektion eine automatische Flußumleitung erfolgt.

Das in den Konzentratbehältern 10 und 12 befindliche Konzentrat kann in flüssiger als auch in fester Form vorliegen.

Da die korrekte Zugabe der Konzentrate automatisch erfolgt, beschränkt sich die Aufgabe des Benutzers auf das Einlegen der Konzentratbehältnisse 10, 12 in eine entsprechende Aufnahmevorrichtung. Die zeitliche Abfolge sowie die Konnektion und die Flußregulierung erfolgen automatisch. Während bei vorbekannten Lösungen einer Flußregulierung durch Drehventile, durch Schlauchquetschventile oder auch durch Sitzventile erfolgen muß, die in ihrem Aufbau aufwendig sind und die ein Fehlbedienungsrisiko durch den Benutzer bedingen, kommt die vorliegende Erfindung ohne derartige Hilfsmittel aus. Ausreichend sind Konnektoren, die zum gewünschten Zeitpunkt eine automatische Konnektierung des zweiten Konzentratbehältnisses 12 mit der Lösungsmittelquelle 60 bzw. mit dem Sammelbehältnis 40 sicherstellen.

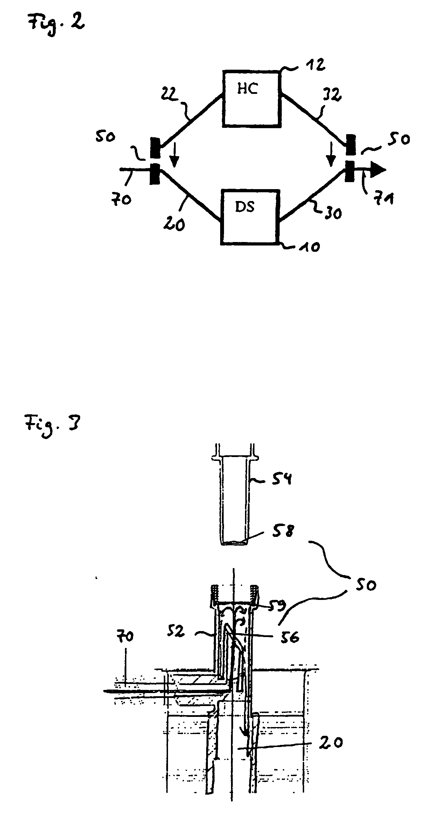

Fig. 2 zeigt nochmals die schematische Darstellung des Flußes des Lösungsmittels durch das erste Konzentratbehältnis 10 unmittelbar vor Konnektierung des zweiten Konzentratbehältnisses 12. Beide Konnektoren 50 werden zum vorgegebenen Zeitpunkt in ihre zweite Position bewegt, in der nunmehr das zweite Konzentratbehältis 12 durchströmt wird.

Fig. 3 zeigt eine Detaildarstellung einer Ausführungsform eines erfindungsgemäßen Konnektors 50, der von einer ersten in eine zweiten Position bewegt werden kann. Der Konnektor 50 weist ein erstes Konnektorelement 52 auf, das mit dem ersten Konzentratbehältnis 10 in Verbindung steht. Ein zweites Konnektorelement 54, das in das erste Konnektorelement 52 einführbar ist, steht mit dem zweiten Konzentratbehältnis 12 in Verbindung. In dem ersten Konnektorelement 52 ist ein Aufstoßdorn 56 vorgesehen, mittels dessen bei der Bewegung des Konnektors 50 in die zweiten Positon eine das zweite Konnektorelement 54 verschließende Membran 58 durchtrennbar ist. Das erste Konnektorelement 52 ist mit einer Silikonmembran 59 verschlossen. In der in Fig. 3 dargestellten Position wird Lösungsmittel durch die Anschlußleitung 70 und durch den Aufstoßdorn 56 schließlich in die Zufuhrleitung 20 des ersten Konzentratbehältnisses 10 bzw. in umgekehrter Richtung durch eine entsprechende Abfuhrleitung und durch den Aufstoßdorn in eine entsprechende Anschlußleitung geleitet. Zu einem vorgegebenen Zeitpunkt wird in den zweiten Konnektionszustand geschaltet, wobei zunächst die Silikonmembran 59 durchtrennt wird, woraufhin eine dichte Verbindung zum zweiten Konzentratbehältnis 12 hergestellt wird. Bei der weiteren Zusammenführung wird die Membran 58 mittels des Aufstoßdorns 56 durchtrennt und das zweite Konnektorelement 54 in den sich um den Aufstoßdorn 56 erstreckenden Ringraum eingeführt. Das Lösungsmittel wird nun durch die Anschlußleitung 70, den Aufstoßdorn 56 und das zweite Konnektorelement 54 in die entsprechende Zufuhrleitung des zweiten Konzentratbehältnisses 12 geführt. Die das zweite Konzentratbehältnis 12 verlassende Lösung wird entsprechend in entgegengesetzter Richtung geführt.

Der oben beschriebene Verbindungsvorgang der Konnektorelemente 52, 54 vollzieht sich auf der Zulauf- sowie auf der Ablaufseite. Vorzugsweise ist das erste Konzentratbehältnis 10 mit zwei ersten Konnektorelementen 52 und das zweite Konzentratbehältnis 12 mit zwei zweiten Konnektorelementen 54 versehen. Zum Zeitpunkt der Flußumschaltung werden nunmehr beide Konnektoren 50 betätigt, d. h. sowohl auf der Zu- als auch auf der Ablaufseite werden die Konnektorelemente 52, 54 zusammengeführt, so daß nunmehr das zweite Konzentratbehältnis 12 durchströmt wird.

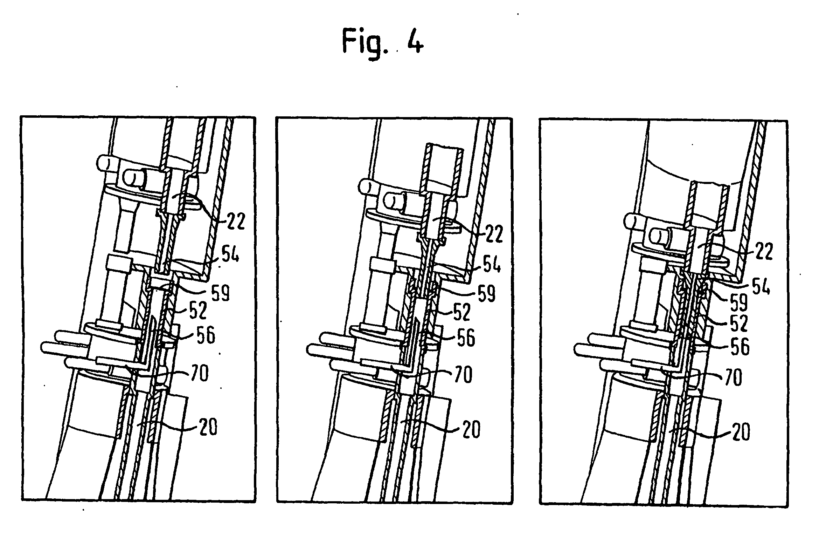

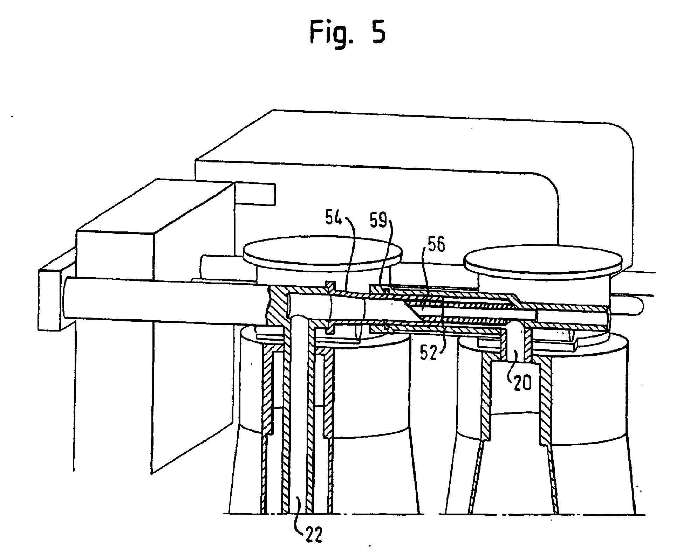

Der soeben geschilderte Konnektionsvorgang ist nochmals in Fig. 4 und Fig. 5 dargestellt. Fig. 4 zeigt eine entsprechende Konnektierung eines ersten und eines zweiten Konzentratbehältnisses in vertikaler Anordnung und Fig. 5 in horizontaler Anordnung. Fig. 4, linke Darstellung zeigt den Konnektor 50 in einer ersten Position, in der die Lösungsmittelquelle über die Anschlußleitung 70, den Aufstoßdorn 56 und die Zufuhrleitung 20 mit dem ersten Konzentratbehältnis in Verbindung steht.
Fig. 4, mittlere Darstellung zeigt den Konnektionszustand, bei dem bereits eine dichtende Verbindung zum zweiten Konzentratbehältnis hergestellt ist, indem das zweite Konnektorelement 54 nach Durchtrennung der Membran 59 in das erste Konnektorelement 52 eingeführt ist. Aus der in Fig. 4, rechte Darstellung gezeigten Position ergibt sich, daß der Aufstoßdorn 56 die das zweite Konnektorelement 54 verschließende Membran 58 (siehe Fig. 3) durchtrennt hat, wodurch eine fluiddichte Verbindung zum zweiten Konzentratbehältnis hergestellt ist. Das Lösungsmittel strömt nun durch die Zufuhrleitung 22 in das zweite Konzentratbehältnis.

## Patentansprüche

1. Verfahren zur Zubereitung von aus wenigstens zwei Konzentraten und einem Lösungsmittel herzustellenden Lösungen, bei dem nach dem Einlegen von Konzentratbehältnissen (10, 12) in zugehörigen Aufnahmen die Verbindung eines ersten Konzentratbehältnisses (10) mit einer Lösungsmittelquelle (60) erfolgt, das erste Konzentratbehältnis (10) von Lösungsmittel durchströmt und die Lösung in ein Sammelbehältnis (40) geführt wird, und bei dem nach dem Ausspülen des Konzentrats aus dem ersten Konzentratbehältnis (10) eine automatische Konnektierung eines zweiten Konzentratbehältnisses (12) erfolgt, aufgrund derer eine automatische Flußumleitung erfolgt, woraufhin das zweite Konzentratbehältnis (12) von Lösungsmittel durchströmt und die Lösung ebenfalls dem Sammelbehältnis (40) zugeführt wird.

2. Verfahren nach Anspruch 1, **dadurch gekennzeichnet, daß** das Lösungsmittel durch Reversosmose hergestelltes hochreines Wasser ist und daß in dem ersten Konzentratbehältnis (10) ein NaCl, NaHCO₃ und Glukose enthaltendes Trockenkonzentrat und in dem zweiten Konzentratbehältnis (12) ein flüssiges Konzentrat vorliegt, das als Hauptbestandteile die Ionen K⁺, Ca²⁺, Mg²⁺ sowie NaCl enthält.

3. Verfahren nach Anspruch 1 oder 2, **dadurch gekennzeichnet, daß** die automatische Konnektierung des zweiten Konzentratbehältnisses (12) erfolgt, wenn eine definierte Zeitspanne seit Beginn der Durchströmung des ersten Konzentratbehältnisses (10) abgelaufen ist, wenn die Leitfähigkeit der das erste Konzentratbehältnis (10) verlassenden Lösung einen Grenzwert unterschritten hat oder wenn der Füllstand in dem Sammelbehältnis (40) einen vorgegeben Wert übersteigt.

4. Vorrichtung zur Zubereitung von aus wenigstens zwei Konzentraten und einem Lösungsmittel herzustellenden Lösungen mit wenigstens zwei Konzentratbehältnissen (10, 12), mit Zufuhrleitungen (20, 22), durch die das Lösungsmittel zu den Konzentratbehältnissen (10, 12) führbar ist, mit Ablaufleitungen (30, 32), mittels derer die Lösungen von den Konzentratbehältnissen (10, 12) in ein Sammelbehältnis (40) führbar sind, mit einem Konnektor (50), der in einer ersten Position eine Lösungsmittelquelle (60) mit dem ersten Konzentratbehältnis (10) und in einer zweiten Position die Lösungsmittelquelle (60) mit dem zweiten Konzentratbehältnis (12) verbindet, sowie mit Mitteln, durch die der Konnektor (50) von der ersten in die zweite Position bewegbar ist, um das zweite Konzentratbehältnis zu konnektieren, wobei aufgrund der Konnektion des zweiten Konzentratbehältnisses eine automatische Flußumleitung erfolgt.

5. Vorrichtung nach Anspruch 4, **dadurch gekennzeichnet, daß** die Konzentratbehältnisse (10, 12) als Einmalartikel ausgeführt sind und mit jeweils zwei selbstdichtenden Konnektorelementen (52, 54) versehen sind.

6. Vorrichtung nach Anspruch 4 oder 5, **dadurch gekennzeichnet, daß** die Konzentratbehältnisse (10, 12) trichterförmig ausgeführt sind.

7. Vorrichtung nach einem der Ansprüche 4 bis 6, **dadurch gekennzeichnet, daß** der Konnektor (50) ein mit einem ersten Konzentratbehältnis (10) in Verbindung stehendes erstes Konnektorelement (52) aufweist, in das ein mit einem zweiten Konzentratbehältnis (12) in Verbindung stehendes zweites Konnektorelement (54) einführbar ist und daß in dem ersten Konnektorelement (52) ein Aufstoßdorn (56) vorgesehen ist, mittels dessen bei der Bewegung des Konnektors (50) in die zweiten Position eine das zweite Konnektorelement (54) verschließende Membran (58) durchtrennbar ist.

8. Vorrichtung nach Anspruch 7, **dadurch gekennzeichnet, daß** eine Anschlußleitung (70, 71) vorgesehen ist, die an dem ersten Konnektorteil (52) angeordnet ist und durch die das Lösungsmittel von der Lösungsmittelquelle (60) zum Konnektor (50) bzw. die Lösung vom Konnektor (50) zum Sammelbehältnis (40) führbar ist.

9. Vorrichtung nach Anspruch 7 oder 8, **dadurch gekennzeichnet, daß** sich um den Aufstoßdorn (56) des ersten Konnektorteils (52) ein Ringraum erstreckt, der einen Teil der Zufuhrleitung (20) bzw. Ablaufleitung (30) bildet, die den Konnektor (50) mit dem ersten Konzentratbehältnis (10) verbindet.

10. Konnektor (50) mit einem ersten Konnektorelement (52), das mit einem ersten Konzentratbehältnis (10) und einer Anschlußleitung (70, 71) in Verbindung steht oder verbindbar ist, und mit einem zweiten Konnektorelement (54), das mit einem zweiten Konzentratbehältnis (12) in Verbindung steht oder verbindbar ist, wobei das erste Konnektorelement (52) von einer Membran (59) verschlossen ist, die durch das in das erste Konnektorelement (52) einführbare zweite Konnektorelement (54) durchtrennbar ist und
wobei das erste Konnektorelement (52) einen hohl ausgeführten Aufstoßdorn (56) aufweist, dessen Innenraum mit einer Anschlußleitung (70, 71) in Verbindung steht oder verbindbar ist und um den sich ein mit einem ersten Konzentratbehältnis (10) in Verbindung stehender oder verbindbarer Ringraum erstreckt,
wobei mittels des Aufstoßdorns (56) eine das zweite Konnektorelement (54) verschließende Membran (58) durchtrennbar ist.

11. Konzentratbehältereinheit mit einem in einem ersten und einem zweiten Konzentratbehältnis (10,12) aufgenommenen festen oder flüssigen Konzentrat, wobei das erste Konzentratbehältnis (10) mit zwei ersten Konnektorelementen (52) und das zweite Konzentratbehältnis (12) mit zwei zweiten Konnektorelementen (54) versehen sind und wobei die ersten Konnektorelemente (52) jeweils eine diese verschließende Membran (59) sowie einen mit einer Anschlußleitung (70, 71) in Verbindung stehenden Aufstoßdorn (56) aufweisen, der von einem mit dem Konzentratbehältnis (10, 12) in Verbindung stehenden Ringraum umgeben ist und wobei die zweiten Konnektorelemente (54) jeweils mit dem Konzentratbehältnis (10, 12) in Verbindung stehen und eine die zweiten Konnektorelemente (54) verschließende Membran (58) aufweisen.

12. Konzentratbehältereinheit nach Anspruch 11, **dadurch gekennzeichnet, daß** diese eine Zufuhr- (20, 22) sowie eine Ablaufleitung (30, 32) umfaßt, die sich von den ersten (52) oder zweiten Konnektorelementen (54) zu dem Konzentratbehältnis (10, 12) bzw. von diesem zu den ersten (52) oder zweiten Konnektorelementen (54) erstrecken.

13. Konzentratbehältereinheit nach Anspruch 11 oder 12, **dadurch gekennzeichnet, daß** die die ersten Konnektorelemente (52) verschließenden Membranen (58, 59) und/oder die die zweiten Konnektorelemente (54) verschließenden Membranen (58, 59) Silikonmembranen sind.

14. Konzentratbehältereinheit nach einem der Ansprüche 11 bis 13, **dadurch gekennzeichnet, daß** das Konzentrat ein NaCl, NaHCO₃ und Glucose enthaltendes Trockenkonzentrat oder ein flüssiges Konzentrat ist, das als Hauptbestandteile die Ionen K⁺, Ca⁺⁺, Mg⁺⁺ sowie NaCl enthält.

15. Konzentratbehältereinheit nach Anspruch 14, **dadurch gekennzeichnet, daß** das flüssige Konzentrat ferner die Bestandteile Citrat und HCl enthält.

## Claims

1. Method for the preparation of solutions to be prepared from at least two concentrates and a solvent, in which, after concentrate containers (10, 12) have been placed in associated receptacles, a first concentrate container (10) is connected to a solvent source (60), solvent flows through the first concentrate container (10) and the solution is guided into a collection container (40), and in which, after the concentrate has been flushed out of the first concentrate container (10), a second concentrate container (12) is automatically connected, as a result of which the flow is automatically reversed, whereupon solvent flows through the second concentrate container (12) and the solution is likewise fed to the collection container (40).

2. Method according to claim 1, **characterized in that** the solvent is high-purity water prepared by reverse osmosis and **in that** a dry concentrate containing NaCl, NaHCO₃ and glucose is present in the first concentrate container (10) and a liquid concentrate which contains the ions K⁺, Ca²⁺, Mg²⁺ and also NaCl as main constituents is present in the second concentrate container (12).

3. Method according to claim 1 or 2, **characterized in that** the second concentrate container (12) is automatically connected when a defined period of time has passed since the start of the flow through the first concentrate container (10), when the conductivity of the solution leaving the first concentrate container (10) has fallen below a limit value or when the fill level in the collection container (40) exceeds a given value.

4. Device for the preparation of solutions to be prepared from at least two concentrates and a solvent, with at least two concentrate containers (10, 12), with feed lines (20, 22) by which the solvent can be guided to the concentrate containers (10, 12), with outflow lines (30, 32) by means of which the solutions can be guided from the concentrate containers (10, 12) into a collection container (40), with a connector (50) which, in a first position, connects a solvent source (60) to the first concentrate container (10) and, in a second position, connects the solvent source (60) to the second concentrate container (12), and with means by which the connector (50) can be moved from the first position into the second in order to connect the second concentrate container, wherein the flow is automatically reversed as a result of the connection of the second concentrate container.

5. Device according to claim 4, **characterized in that** the concentrate containers (10, 12) are designed as disposable articles and are each provided with two self-sealing connector elements (52, 54).

6. Device according to claim 4 or 5, **characterized in that** the concentrate containers (10, 12) are funnel-shaped.

7. Device according to one of claims 4 to 6, **characterized in that** the connector (50) has a first connector element (52), in communication with a first concentrate container (10), into which a second connector element (54), in communication with a second concentrate container (12), can be introduced, and **in that** a push-open pin (56) is provided in the first connector element (52), by means of which a membrane (58) sealing off the second connector element (54) can be severed when the connector (50) moves into the second position.

8. Device according to claim 7, **characterized in that** a connection line (70, 71) is provided which is attached to the first connector part (52) and by which the solvent can be guided from the solvent source (60) to the connector (50) or the solution can be guided from the connector (50) to the collection container (40).

9. Device according to claim 7 or 8, **characterized in that** an annular space which forms a part of the feed line (20) or outflow line (30) which connects the connector (50) to the first concentrate container (10) extends around the push-open pin (56) of the first connector part (52).

10. Connector (50) with a first connector element (52) which is or can be connected to a first concentrate container (10) and a connection line (70, 71), and with a second connector element (54) which is or can be connected to a second concentrate container (12),
wherein the first connector element (52) is sealed off by a membrane (59) which can be severed by the second connector element (54) which can be introduced into the first connector element (52) and
wherein the first connector element (52) has a hollow push-open pin (56), the inside of which is or can be connected to a connection line (70, 71) and around which an annular space which is or can be connected to a first concentrate container (10) extends,
wherein a membrane (58) sealing off the second connector element (54) can be severed by means of the push-open pin (56).

11. Concentrate container unit, with a solid or liquid concentrate accommodated in a first and a second concentrate container (10, 12), wherein the first concentrate container (10) is provided with two first connector elements (52) and the second concentrate container (12) with two second connector elements (54) and wherein in each case the first connector elements (52) have a membrane (59) sealing off the latter and a push-open pin (56), in communication with a connection line (70, 71), which is surrounded by an annular space in communication with the concentrate container (10, 12), and wherein in each case the second connector elements (54) are in communication with the concentrate container (10, 12) and have a membrane (58) sealing off the second connector elements (54).

12. Concentrate container unit according to claim 11, **characterized in that** this comprises a feed line (20, 22) and a outflow line (30, 32) which extend from the first (52) or second (54) connector elements to the concentrate container (10, 12) or from the latter to the first (52) or second (54) connector elements.

13. Concentrate container unit according to claim 11 or 12, **characterized in that** the membranes (58, 59) sealing off the first connector elements (52) and/or the membranes (58, 59) sealing off the second connector elements (54) are silicone membranes.

14. Concentrate container unit according to one of claims 11 to 13, **characterized in that** the concentrate is a dry concentrate containing NaCl, NaHCO₃ and glucose or a liquid concentrate which contains the ions K⁺, Ca⁺⁺, Mg** and also NaCl as main constituents.

15. Concentrate container unit according to claim 14, **characterized in that** the liquid concentrate furthermore contains the constituents citrate and HCl.

## Revendications

1. Procédé destiné à la préparation de solutions à réaliser à partir d'au moins deux concentrés et un agent de dissolution, dans lequel, après la mise en place des récipients de concentré (10, 12) dans des dispositifs de réception appropriés, est établie la liaison d'un premier récipient de concentré (10) avec une source d'agent de dissolution (60), l'agent de dissolution circule à travers le premier récipient de concentré (10) et la solution est acheminée dans un collecteur (40), et dans lequel, après l'évacuation du concentré hors du premier récipient de concentré (10), il se produit un branchement automatique d'un deuxième récipient de concentré (12), en raison duquel se produit une déviation automatique du flux, à la suite de quoi l'agent de dissolution circule à travers le deuxième récipient de concentré (12) et la solution est également acheminée vers le collecteur (40).

2. Procédé selon la revendication 1, **caractérisé en ce que** l'agent de dissolution est de l'eau très pure, obtenue par osmose inverse, et **en ce que** le premier récipient de concentré (10) contient un concentré sec, contenant du NaCl, NaHCO₃ et du glucose, et le deuxième récipient de concentré (12) contient un concentré liquide, dont les composants principaux sont les ions K⁺, Ca²⁺, Mg²⁺, ainsi que NaCl.

3. Procédé selon la revendication 1 ou 2, **caractérisé en ce que** le branchement automatique du deuxième récipient de concentré (12) est effectué lorsqu'un intervalle de temps défini s'est écoulé depuis le début du passage du flux à travers le premier récipient de concentré (10), lorsque la conductivité de la solution sortant du premier récipient de concentré (10) est inférieure à une valeur limite ou lorsque le niveau de remplissage dans le collecteur (40) est supérieur à une valeur prédéfinie.

4. Dispositif destiné à la préparation de solutions à réaliser à partir d'au moins deux concentrés et un agent de dissolution, comportant au moins deux récipients de concentré (10, 12), des conduites d'acheminement (20, 22), par lesquelles l'agent de dissolution peut être acheminé vers les récipients de concentré (10, 12), des conduites d'évacuation (30, 32), par lesquelles les solutions peuvent être acheminées depuis les récipients de concentré (10, 12) vers un collecteur (40), un raccord (50) qui, dans une première position, fait communiquer une source d'agent de dissolution (60) avec le premier récipient de concentré (10) et, dans une deuxième position, fait communiquer la source d'agent de dissolution (60) avec le deuxième récipient de concentré (12), ainsi que des moyens permettant de déplacer le raccord (50) de la première position dans la deuxième position pour le branchement du deuxième récipient de concentré, sachant que, compte tenu du branchement du deuxième récipient de concentré, il se produit une déviation automatique du flux.

5. Dispositif selon la revendication 4, **caractérisé en ce que** les récipients de concentré (10, 12) sont réalisés sous forme de produits jetables et sont munis chacun de deux éléments de raccord (52, 54) auto-étanches.

6. Dispositif selon la revendication 4 ou 5,
**caractérisé en ce que** les récipients de concentré (10, 12) sont réalisés en forme d'entonnoir.

7. Dispositif selon l'une quelconque des revendications 4 à 6, **caractérisé en ce que** le raccord (50) comporte un premier élément de raccord (52), qui est relié au premier récipient de concentré (10) et dans lequel peut être enfiché un deuxième élément de raccord (54) relié à un deuxième récipient de concentré (12), et **en ce qu'**une broche de perçage (56) est prévue dans le premier élément de raccord (52), laquelle permet de transpercer une membrane (58) obturant le deuxième élément de raccord (54), lorsque le raccord (50) est déplacé dans la deuxième position.

8. Dispositif selon la revendication 7, **caractérisé en ce qu'**il est prévu une conduite de jonction (70, 71), qui est disposée au niveau du premier élément de raccord (52) et à travers laquelle l'agent de dissolution peut être acheminé depuis une source d'agent de dissolution (60) vers le raccord (50) ou la solution peut être acheminée depuis le raccord (50) vers le collecteur (40).

9. Dispositif selon la revendication 7 ou 8, **caractérisé en ce qu'**autour de la broche de perçage (56) du premier élément de raccord (52) s'étend une chambre annulaire, qui forme une partie de la conduite d'admission (20) ou de la conduite d'évacuation (30), qui relie le raccord (50) avec le premier récipient de concentré (10).

10. Raccord (50) comportant un premier élément de raccord (52), qui est relié ou peut être relié à un premier récipient de concentré (10) et une conduite de jonction (70, 71), et comportant un deuxième élément de raccord (54) qui est relié ou peut être relié à un deuxième récipient de concentré (12),
dans lequel le premier élément de raccord (52) est obturé par une membrane (59), qui peut être transpercée par le deuxième élément de raccord (54), propre à être introduit dans le premier élément de raccord (52), et
dans lequel le premier élément de raccord (52) comporte une broche de perçage (56) creuse, dont l'intérieur est relié ou peut être relié à une conduite de jonction (70, 71) et autour de laquelle s'étend une chambre annulaire reliée ou pouvant être reliée à un premier récipient de concentré (10),
dans lequel une membrane (58), obturant le deuxième élément de raccord (54), peut être transpercée par la broche de perçage (56).

11. Unité de récipients de concentré avec un concentré solide ou liquide, contenu dans un premier et un deuxième récipient de concentré (10, 12), le premier récipient de concentré (10) étant muni de deux premiers éléments de raccord (52) et le deuxième récipient de concentré (12) étant muni de deux deuxièmes éléments de raccord (54), et lesdits premiers éléments de raccord (52) comportant chacun une membrane (59) les obturant et une broche de perçage (56), qui est reliée à une conduite de jonction (70, 71) et qui est entourée par une chambre annulaire reliée au récipient de concentré (10, 12), et les deuxièmes éléments de raccord (54) étant reliés chacun au récipient de concentré (10, 12) et comportant une membrane (58) obturant les deuxièmes éléments de raccord (54).

12. Unité de récipients de concentré selon la revendication 11, **caractérisée en ce que** ladite unité comporte une conduite d'admission (20, 22) et une conduite d'évacuation (30, 32), qui s'étendent depuis les premiers (52) et les deuxièmes éléments de raccord (54) vers le récipient de concentré (10, 12) et respectivement depuis celui-ci vers les premiers (52) et les deuxièmes éléments de raccord (54).

13. Unité de récipients de concentré selon la revendication 11 ou 12, **caractérisée en ce que** les membranes (58, 59) obturant les premiers éléments de raccord (52) et/ou les membranes (58, 59) obturant les deuxièmes éléments de raccord (54) sont des membranes en silicone.

14. Unité de récipients de concentré selon l'une quelconque des revendications 11 à 13, **caractérisée en ce que** le concentré est un concentré sec, contenant du NaCl, NaHCO₃ et du glucose, ou un concentré liquide, dont les composants principaux sont les ions K⁺, Ca²⁺, Mg²⁺, ainsi que NaCl.

15. Unité de récipients de concentré selon la revendication 14, **caractérisée en ce que** le concentré liquide contient en outre les composants citrate et HCl.
